# EUROPEAN PATENT SPECIFICATION

(11) **EP 1 917 058 B1**
(45) Date of publication and mention of the grant of the patent: **23.10.2013**
(21) Application number: 06751530.4
(22) Date of filing: 28.04.2006
(51) Int. Cl.: A61M 16/04

(54) **MULTILUMEN TRACHEAL CATHETER WITH RINSE LUMEN**
MULTILUMEN-TRACHEALKATHETER MIT SPÜLLUMEN
CATHETER TRACHEAL A LUMIERES MULTIPLES ET LUMIERE DE RINÇAGE

(30) Priority: 25.08.2005 US 211267
(43) Date of publication of application: 07.05.2008
(73) Proprietor: KIMBERLY-CLARK WORLDWIDE, INC., Neenah, WI 54956 (US)
(72) Inventor: MADSEN, Edward, B., Riverton, Utah 80465 (US); TEIXEIRA, Scott, M., Cumming, Georgia 30040 (US)
(74) Representative: Mabey, Katherine Frances
(86) International application number: PCT/US2006/015861
(87) International publication number: WO 2007/024288

(56) References cited:
- WO-A-93/09833
- WO-A-99/38548
- WO-A1-93/17744
- US-A- 4 881 542
- US-A- 4 976 261
- US-A- 5 687 714
- US-A- 5 803 078
- US-A- 5 819 723
- US-A1- 2002 014 238

## Description

### BACKGROUND

The present invention relates to a tracheal tube used for mechanical ventilation of a hospital patient, by insertion of the tube into the trachea of the patient. In particular, the present invention relates to a tracheal tube having means for irrigating and/or evacuating contaminated secretions accumulating above the tracheal tube cuff and thereby reducing the risk of such contaminated secretions entering the lungs of the patient.

Endotracheal intubation involves the insertion of a tubular device, known as an endotracheal tube, into the trachea of a patient. The endotracheal tube passes through the trachea and terminates at a position above the carina, anterior to a position between the second and fourth thoracic vertebrate. Gases may then be introduced through the endotracheal tube and into the lungs of the patient.

The primary purposes of endotracheal intubation are to mechanically ventilate the patient's lungs when a disease prevents the patient from normal, breathing induced ventilation, or to apply anesthetic gases during surgical intervention. In order to create enough air pressure to accomplish such mechanical ventilation and to prevent escape of gases past the tube, it is necessary to seal the passageway around the endotracheal tube. A seal may be produced by the use of an inflatable cuff formed integrally with and surrounding the endotracheal tube. When the endotracheal tube has been introduced into the patient's trachea, the inflatable cuff will normally be located about 3 to 5 centimeters above the carina and within the tube-like trachea.

The inflatable cuff is then inflated so as to engage the wall of the trachea and thereby seal the trachea and prevent gases being introduced through the tracheal tube from simply backing up around the tube. While treatment of this sort has proved successful for patients having chronic or acute respiratory diseases, there is a constant risk of several complications.

In particular, many patients receiving endotracheal intubation develop pneumonia, resulting from an infection of the lungs, possibly induced by contaminated, pooled secretions entering the trachea and the lungs after bypassing the epiglottis during intubation. The epiglottis normally operates as a valve which selectively closes the entry into the trachea and lungs, to prevent the introduction of secretions and particulate matter. However, when a tracheal tube is in place, the epiglottis is held in an open position, and secretions which would normally be directed away from the trachea and into the digestive system, instead follow the path of the endotracheal tube and pool above the inflatable cuff of the endotracheal tube.

The greatest risk of such infectious secretions reaching the lungs is upon the cessation of mechanical ventilation. In particular, when the need for endotracheal intubation ends, the inflatable cuff of the endotracheal tube is deflated so that the endotracheal tube may be withdrawn from the patient. The infectious secretions which have pooled above the inflatable cuff are then released and are free to flow into the lungs, where bronchitis or pneumonia may rapidly develop. There is also the risk of the infectious secretions reaching the lungs during the intubation, by aspiration of the secretions past the tracheal tube cuff.

To overcome these risks, it is known in the prior art to combine a single lumen suction tube with a tracheal tube. The suction tube is joined to the endotracheal tube in a suitable manner, the end of the suction tube terminating at a position above the inflatable cuff. The suction tube provides means for suction or evacuation of any pooled secretions which accumulate in the trachea above the inflatable cuff. However, such prior art devices have the disadvantage that use of a single lumen for the suction tube often causes direct suction to be exerted on the tracheal mucosa which may then result in damage to the mucosa.

U.S. Pat. No. 4,840,173 to Porter III, describes an endotracheal tube having a single lumen suction tube merged thereto. In particular, this patent describes a device wherein the suction tube is laminated to the outside of the ventilation tube, so that the suction tube terminates at a position just above the inflatable cuff. The suction tube includes multiple openings which may be used to evacuate secretions which pool above the inflatable cuff. In addition, the inflatable cuff includes a section immediately adjacent to the end of the suction tube that is less flexible than the rest of the inflatable cuff, to insure that the flexible material of the inflatable cuff is not sucked up against the suction tube openings. The endotracheal tube described in the Porter III patent has the disadvantages noted above, that the single lumen suction tube may exert suction on the tracheal mucosa and thereby cause damage to the mucosa. Further, the Porter III device is of a relatively complex design, requiring difficult processing, resulting in expensive production.

U.S. Pat. No. 5,143,062, issued to Peckham, discloses an endotracheal tube comprising a double lumen through which air may be circulated, creating an indirect gentle suction through a suction eye communicating with the distal ends of the lumens, and located at a position proximal to the inflation cuff. This design, however, does not provide adequate suction necessary for aspirating secretions and is easily occluded.

WO93/1774 discloses an endotracheal tube having aerosol generating means.

In fact, one problem that frequently arises in many of these catheters is that the suction port becomes occluded with secretions, rendering the function unusable. As such, what is needed is a multilumen catheter capable of suctioning secretions which have pooled above the inflatable cuff in a manner sufficient to accomplish the task but not so strong so as to cause damage to the mucosa. The suction function on such a device would be capable of being cleaned of accumulated secretions, preferably while in use. The instant invention addresses these problems by providing a multilumen tracheal tube and suction catheter system with a rinse function.

### SUMMARY OF THE INVENTION

The present invention provides a tracheal tube in accordance with claim 1.

The present invention improves upon a tracheal tube by incorporating a rinse lumen therein. In one embodiment, the tracheal tube is formed from a flexible cannula having a length, a distal end, and a proximal end. The cannula consists of a plurality of walls extending substantially along the length of the cannula, dividing the cannula into a plurality of separate lumens including a respiratory lumen, a suction lumen, a rinse lumen, and an inflation lumen. An inflatable cuff surrounds the cannula proximal to the distal end. The inflatable cuff is adapted to seal the trachea of a patient. The inflation lumen is in fluid communication with the inflatable cuff. A port extends through a side wall of the cannula proximal to the inflatable cuff. The port is in fluid communication with the suction lumen. The rinse lumen may terminate within the suction lumen proximal to the port or may terminate within a chamber formed within the suction lumen, the chamber being proximate to the port.

The tracheal tube has a plurality of suction lumens and a plurality of rinse lumens. Each suction lumen terminates in a port and each rinse lumen terminates within one of the suction lumens proximal to one of the ports. There may also be a plurality of rinse lumens for each suction lumen. A rinse liquid is adapted to be flushed through the rinse lumen and extracted via the suction lumen.

The inflatable cuff may have a shape to block a trachea beneath a glottis of the patient. The port may enable suctioning of a subglottic space external to the cannula while simultaneously enabling ventilation through the respiratory lumen.

In any of the embodiments the tracheal tube may have a low profile extension upon an exterior surface of the cannula at the port to extend the effective reach of the suction lumen. The rinse lumen may terminate within the low profile extension.

A method of suctioning fluids from the subglottic space within an intubated patient is described. The method includes inserting a multilumen catheter into a patient's trachea, and inflating a cuff so as to sealingly engage the walls of the trachea to minimize the flow of fluids from the subglottic space into the patients lungs. The patient may be continuously ventilated through at least one lumen of the multilumen catheter. Suctioning of fluids from the subglottic space may be conducted through at least one other lumen. This lumen should have a port extending through a side wall of the catheter proximate to the cuff to access such fluids. This suction lumen may be rinsed by introducing a rinse liquid into the suction lumen proximate to the port through at least another lumen while suctioning fluids from the subglottic space. Rinsing may be accomplished under turbulent flow conditions, including as a spray.

Other objects, advantages and applications of the present invention will be made clear by the following detailed description of a preferred embodiment of the invention and the accompanying drawings wherein reference numerals refer to like or equivalent structures.

### BRIEF DESCRIPTION OF THE DRAWINGS

FIG. 1 is an elevational view of one embodiment of a multilumen catheter not in accordance with the present invention;
FIG. 2 is a cross-sectional view of the FIG. 1 catheter at through line 2-2;
FIG. 3 is a cross-sectional view of the FIG. 1 catheter taken longitudinally through the catheter at the port region;
FIG. 4 is a cross-sectional view of an alternative embodiment of the FIG. 1 catheter, depicting the chamber; and
FIG. 5 is a cross-sectional view of yet another embodiment of the FIG. 1 catheter, depicting an optional low profile extension.

### DETAILED DESCRIPTION

Reference will now be made to the drawings in which the various elements of the present invention will be given numeral designations and in which the invention will be discussed so as to enable one skilled in the art to make and use the invention. It is to be understood that the following description is only exemplary of the principles of the present invention, and should not be viewed as narrowing the pending claims. Those skilled in the art will appreciate that aspects of the various embodiments discussed may be interchanged and modified without departing from the scope of the invention.

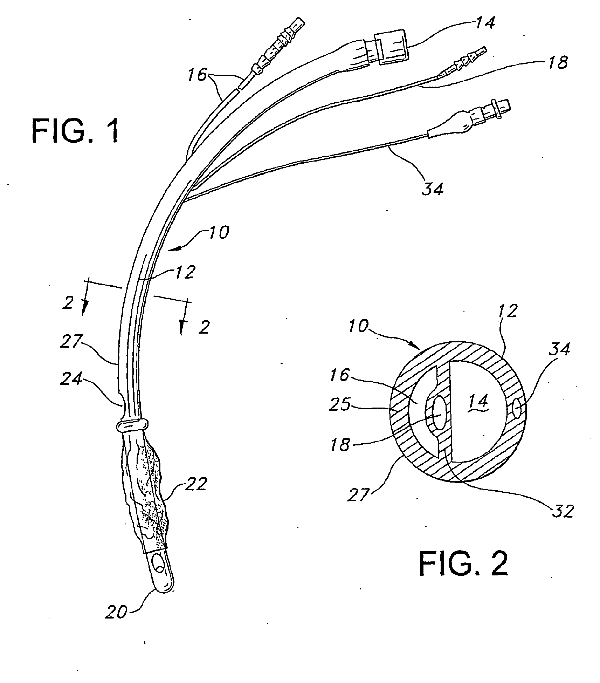

Referring to FIGs. 1 and 2, a tracheal tube 10 is depicted. The tracheal tube 10 in the depicted embodiment is a multilumen cannula 12 having at least one respiratory lumen 14, at least one suction lumen 16, and at least one rinse lumen 18. In the embodiment, each of these lumens is at least partially internal to the cannula 12. The respiratory lumen 14 extends through the entire cannula 12 and is adapted to mechanically ventilate a patient (not shown). As such, a distal end 20 of the cannula 12 is situated within the upper respiratory system of the patient. A balloon, bladder, or inflatable cuff 22 is provided proximal to the distal end 20. The cuff 22 is shaped so that when inflated, it blocks the patient's trachea beneath the glottal area. This is known and understood by those skilled in the art to eliminate or at least to minimize the undesirable flow of fluids from the glottal and subglottal regions of the patient into the bronchus and lungs of the patient.

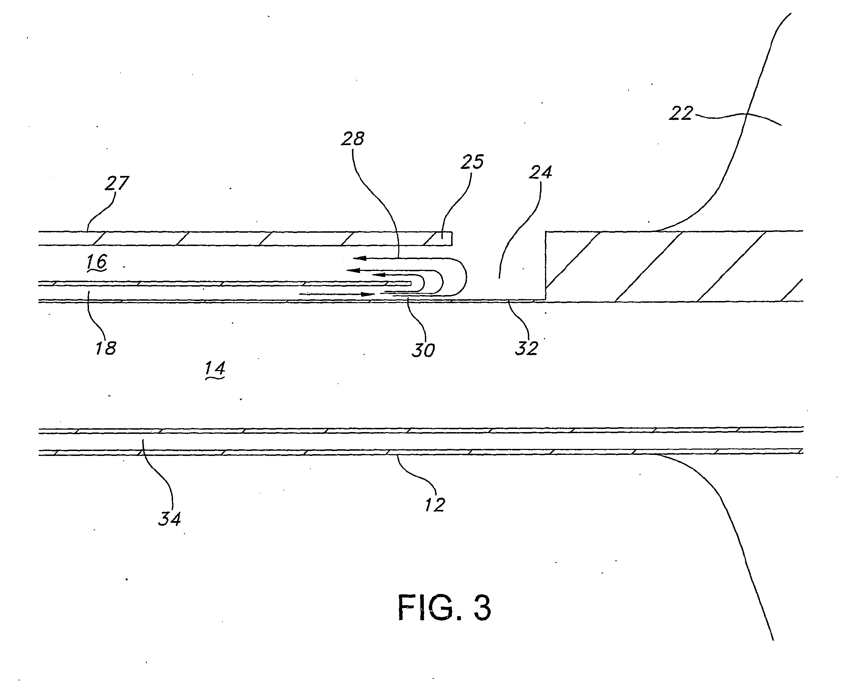

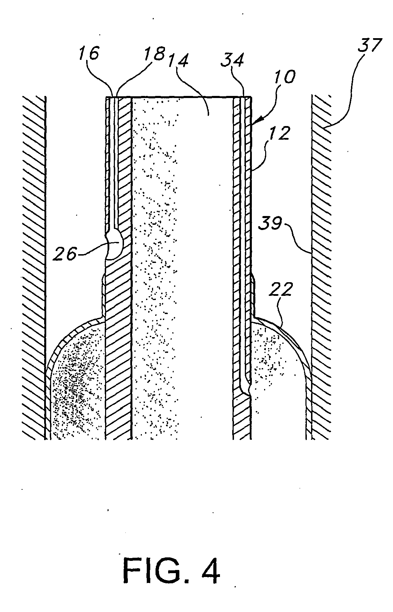

A port 24 extends from the suction lumen 16 through a wall 25 of the cannula 12 to an exterior surface 27 of the cannula 12. The port 24 in the depicted embodiment is proximate to an upper surface of the cuff 22. As such, the suction lumen 16 is adapted to suction fluids that collect above the cuff 22 in the patient's subglottic area without negatively impacting ventilation of the patient through the respiratory lumen 14. The rinse lumen 18, in this embodiment terminates within the cannula 12, specifically within the suction lumen 16 at an exit 30 as depicted in FIG. 3. Moreover, as depicted in FIG. 3, the rinse lumen 18 may terminate proximate to the port 24; or within a chamber 26 proximate to the port 24 as depicted in FIG. 4. In either case, the rinse lumen 18 provides a path for the introduction of a rinse liquid 28. This is depicted in FIG. 3 as a spray pattern. The rinse liquid 28 is introduced into the suction lumen 16 while the suction lumen is suctioning or otherwise evacuating the subglottic space. This is performed at the discretion of the caregiver in order to clean secretions and other liquids that may collect and potentially clog the suction lumen 16. In the embodiments depicted in each of FIGs. 1 through 4, the rinse lumen 18 is situated so as to contain the rinse liquid 28 within the suction lumen 16 and be suctioned along with the pooled liquids and other potentially clogging secretions contained within the suction lumen 16.

The rinse liquid 28 may comprise water, saline, as well as some other biocompatible liquid. A medicament, for example, an antiseptic or an antibiotic, or a treatment such as a surfactant may be added to the rinse liquid to obtain a desired effect on the patient, or to ease suctioning and/or cleaning of the suction lumen 16. Since the main purpose of the rinse liquid 28 is to rinse and/or clean the suction lumen 16, introducing the liquid into the suction lumen 16 in a turbulent manner will enable better cleaning of the suction lumen. As such the exit 30 of the rinse lumen 18 may be configured so as to foster turbulent flow or a spray pattern as depicted in FIG. 3. Moreover the shape of the chamber 26 if existent may contribute to such turbulence or provide a volume within which a spray may desirably be directed as depicted in FIG. 4.

Looking back once again to the cross sectional view of FIG. 2, one possible configuration of the tracheal tube 10 is depicted, more specifically a potential lumen arrangement is depicted within the cannula 12. As can be seen, the respiratory lumen 14 is separated from the suction lumen 16 by an internal wall 32 that extends substantially along the entire length of the cannula 12. Formed into the internal wall 32 is the rinse lumen 18 which as described above terminates or exits at exit 30 within the suction lumen 16 or within the chamber 26, either being proximate to the port 24. This configuration is of course only meant to suggest one possible arrangement. Other arrangements are included in the scope of the invention. For example, the layout of the lumens within the cannula 12 may be altered, moreover, the rinse lumen 18 may be formed in another wall, such as wall 25 of the cannula or it may be a self contained lumen not embedded within any one of the walls of the cannula 12. FIGs. 2, 3 and 4 also depict an inflation lumen 34. The inflation lumen 34 is in fluid communication with the inflatable cuff 22 and as such controls inflation and deflation of the cuff 22 as would be understood by those of skill in the art. FIG. 4 depicts the tracheal tube 10 in position, that is, with the balloon 22 seated against the tracheal mucosa or tracheal wall 39 of the patient's trachea 37.

In embodiments of the invention, a plurality of suction lumens 16 are provided. Each suction lumen is configured essentially as described above, in that each is rinsed by a rinse liquid exiting a rinse lumen 18. A dedicated rinse lumen 18 may be provided for each suction lumen 16. The arrangement of lumens within the cannula 12 is not limited in scope to any particular configuration. Of course in each of the embodiments described more than one rinse lumen 18 may be provided for any one suction lumen 16. Such an arrangement may prove beneficial in more thorough rinsing of the suction lumen or lumens. Any of these embodiments are easily understood by one of skill in the art as they merely increase the number and arrangement of lumens provided. As such no specific drawings are needed for an understanding of these variations.

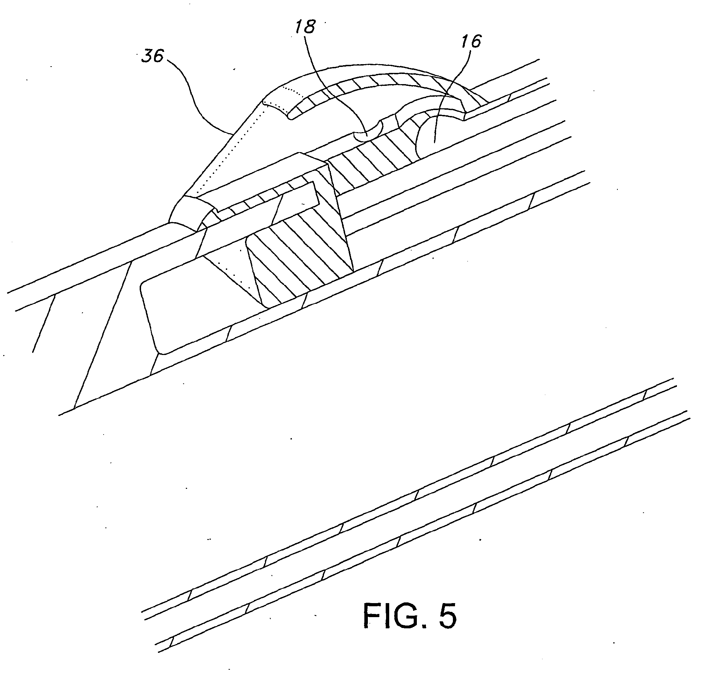

Yet in another embodiment, as shown in FIG. 5, a low profile extension 36 may be provided on the wall 25 of the cannula 12 such that it overlaps the port 24 and it extends the effective reach of the suctioning capabilities radially outward a distance from the wall 25 of the cannula 12 closer to the tracheal wall of the patient. In such configurations the exit 30 of the rinse lumen 18 may be placed as near as possible, including within the low profile extension 36 so as to more effectively rinse the suction lumen 16 and extension 36. Such an extension is disclosed in co-pending US Patent Application filed by the same inventors on August 25, 2005 under Kimberly-Clark Docket Number 21852 under US Express Mail Number EV094172642US.

In use, the caregiver would insert the multilumen catheter or tracheal tube 10 into the patient's trachea 37 in a manner known and understood by those of skill in the art. The inflatable cuff 22 would be inflated through the inflation lumen 34 so as to sealingly engage the walls 39 of the patient's trachea 37. This would effectively prevent or at least minimize flow of undesirable fluids from the subglottic space into the bronchus and lungs. Ventilation of the patient through the respiratory lumen 14 may occur at this time and continue for as long as necessary. At the discretion of the caregiver, the subglottic space within the patient's trachea may be suctioned through the suction lumen 16 via the port 24 through the wall 25 of the cannula 12. During suction, the suction lumen 16 may be rinsed by introduction of the rinse liquid 28 through the rinse lumen 18. The rinse liquid 28 may be injected into the suction lumen 16 in a turbulent manner, including as a spray at or near the port 24 so as to better rinse the entire lumen 16. Rinsing the suction lumen 16 at the same time that suctioning is performed serves at least two function, the first is that it minimizes the inadvertent flow of the rinse liquid out of the cannula 12 and into the patient's subglottic space, and the second is that it increases the turbulent flow of the rinse liquid at the port and throughout the suction lumen as well. Alternatively, a treatment may be added to the rinse liquid such as a medicament, for example, an antiseptic or an antibiotic. In that case, it may be desirable to allow the rinse liquid to exit the cannula 12 so as to gain the desired therapeutic effect prior to suctioning.

As used herein and in the claims, the term "comprising" is inclusive or open-ended and does not exclude additional unrecited elements, compositional components, or method steps.

While the invention has been described in detail with respect to specific embodiments thereof, it will be apparent to those skilled in the art that various alterations, modifications and other changes may be made to the invention without departing from the scope of the present invention. It is therefore intended that the claims cover all such modifications, alterations and other changes encompassed by the appended claims.

## Claims

1. A tracheal tube (10) comprising:
a flexible cannula (12) having a length, a distal end (20), and a proximal end, **characterised in that** the cannula comprises a plurality of walls extending substantially along the length dividing the cannula into a plurality of separate lumens including a respiratory lumen (14), a suction lumen (16), a rinse lumen (18), and an inflation lumen;
an inflatable cuff (22) surrounding the cannula proximal to the distal end (20), the inflatable cuff adapted to seal the trachea of a patient, the inflation lumen in fluid communication with the inflatable cuff; and
a port (24) extending through a side wall (25) of the cannula (12) to an exterior surface of the cannula (12) proximal to the inflatable cuff (22), the port in fluid communication with the suction lumen (16), the rinse lumen (18) terminating within the suction lumen (16) proximal to the port (24), the tracheal tube comprising a plurality of suction lumens and a plurality of rinse lumens, each suction lumen terminating in a port and each rinse lumen terminating within one of the suction lumens proximal to one of the ports.

2. The tracheal tube of claim 1 comprising a rinse liquid adapted to be flushed through the rinse lumen and extracted via the suction lumen.

3. The tracheal tube of claim 1 comprising a plurality of rinse lumens for each suction lumen.

4. The tracheal tube of claim 1 comprising a low profile extension upon an exterior surface of the cannula at the port to extend the effective reach of the suction lumen.

5. The tracheal tube of claim 1 wherein the rinse lumen comprises a passage formed within the wall (32) disposed between the respiratory lumen and the suction lumen.

6. The tracheal tube of claim 1 wherein the rinse lumen terminates within a chamber formed within the suction lumen, the chamber being proximate to the port.

## Patentansprüche

1. Trachealtubus (10), umfassend:
eine flexible Kanüle (12) mit einer Länge, einem distalen Ende (20) und einem proximalen Ende, **dadurch gekennzeichnet, dass** die Kanüle eine Vielzahl von Wänden umfasst, die sich im Wesentlichen entlang der Länge erstrecken, welche die Kanüle in eine Vielzahl von getrennten Lumina teilt, welche ein Respirationslumen (14), ein Sauglumen (16), ein Spüllumen (18) und ein Inflationslumen beinhalten;
eine inflatierbare Manschette (22), welche die Kanüle proximal des distalen Endes (20) umgibt, wobei die inflatierbare Manschette angepasst ist, die Trachea eines Patienten abzudichten, wobei das Inflationslumen in Fluidkommunikation mit der inflatierbaren Manschette steht; und
einen Anschluss (24), der sich durch eine Seitenwand (25) der Kanüle (12) zu einer äußeren Oberfläche der Kanüle (12) proximal der inflatierbaren Manschette (22) erstreckt, wobei der Anschluss in Fluidkommunikation mit dem Sauglumen (16), steht, wobei das Spüllumen (18) in dem Sauglumen (16) proximal des Anschlusses (24) endet, wobei der Trachealtubus eine Vielzahl von Sauglumina und eine Vielzahl von Spüllumina umfasst, wobei jedes Sauglumen in einem Anschluss endet und wobei jedes Spüllumen in einem der Sauglumina proximal eines der Anschlüsse endet.

2. Trachealtubus gemäß Anspruch 1, welcher eine Spülflüssigkeit umfasst, die geeignet ist, durch das Spüllumen gespült und durch das Sauglumen herausgezogen zu werden.

3. Trachealtubus gemäß Anspruch 1, welcher eine Vielzahl von Spüllumina für jedes Sauglumen umfasst.

4. Trachealtubus gemäß Anspruch 1, welcher eine Verlängerung mit geringem Querschnitt auf einer äußeren Oberfläche der Kanüle bei dem Anschluss umfasst, um die effektive Reichweite des Sauglumens zu verlängern.

5. Trachealtubus gemäß Anspruch 1, wobei das Spüllumen eine Passage umfasst, die in der Wand (32) gebildet ist, die zwischen dem Respirationslumen und dem Sauglumen angeordnet ist.

6. Trachealtubus gemäß Anspruch 1, wobei das Spüllumen in einer Kammer endet, welche in dem Sauglumen gebildet ist, wobei sich die Kammer in der Nähe des Anschlusses befindet.

## Revendications

1. Tube trachéal (10) comprenant :
une canule souple (12) ayant une longueur, une extrémité distale (20) et une extrémité proximale, **caractérisé en ce que** la canule comprend une pluralité de parois s'étendant sensiblement le long de la longueur divisant la canule en une pluralité de lumières séparées incluant une lumière de respiration (14), une lumière d'aspiration (16), une lumière de rinçage (18) et une lumière de gonflage;
un manchon gonflable (22) entourant la canule proximale à l'extrémité distale (20), le manchon gonflable étant adapté pour sceller la trachée d'un patient, la lumière de gonflage étant en communication fluidique avec le manchon gonflable ; et
un orifice (24) s'étendant à travers une paroi latérale (25) de la canule (12) jusqu'à une surface extérieure de la canule (12) proximale au manchon gonflable (22), l'orifice étant en communication fluidique avec la lumière d'aspiration (16), la lumière de rinçage (18) se terminant à l'intérieur de la lumière d'aspiration (16) proximale à l'orifice (24), le tube trachéal comprenant une pluralité de lumières d'aspiration et une pluralité de lumières de rinçage, chaque lumière d'aspiration se terminant dans un orifice et chaque lumière de rinçage se terminant à l'intérieur de l'une des lumières d'aspiration proximales à l'un des orifices.

2. Tube trachéal selon la revendication 1, comprenant un liquide de rinçage adapté pour être chassé à travers la lumière de rinçage et extrait par la lumière d'aspiration.

3. Tube trachéal selon la revendication 1, comprenant une pluralité de lumières de rinçage pour chaque lumière d'aspiration.

4. Tube trachéal selon la revendication 1, comprenant une extension à faible profil sur une surface extérieure de la canule sur l'orifice pour prolonger la portée effective de la lumière d'aspiration.

5. Tube trachéal selon la revendication 1, dans lequel la lumière de rinçage comprend un passage formé à l'intérieur de la paroi (32) disposé entre la lumière de respiration et la lumière d'aspiration.

6. Tube trachéal selon la revendication 1, dans lequel la lumière de rinçage se termine à l'intérieur d'une chambre formée à l'intérieur de la lumière d'aspiration, la chambre étant proximale à l'orifice.
